Europäisches Patentamt

European Patent Office

Office européen des brevets

(19)

(11) Veröffentlichungsnummer: **0 017 924**
A2

(12) # EUROPÄISCHE PATENTANMELDUNG

(21) Anmeldenummer: 80101939.9

(22) Anmeldetag: **10.04.80**

(51) Int. Cl.³: **C 07 C 105/00**

(30) Priorität: **11.04.79 CH 3474/79**

(43) Veröffentlichungstag der Anmeldung: **29.10.80**
Patentblatt 80/22

(84) Benannte Vertragsstaaten: **BE DE FR GB IT LU NL**

(71) Anmelder: **LONZA AG, Gampel/Wallis (CH)**

(72) Erfinder: **Althaus, Hans, Dr., Holzackerweg 6, Glis
(Kanton Wallis) (CH)**

(74) Vertreter: **Weinhold, Peter, Dr. et al, Siegfriedstrasse 8,
D-8000 München 40 (DE)**

(54) Verfahren zur Herstellung von Azoxybenzol durch katalytische Hydrierung von Nitrobenzol mit molekularem Wasserstoff.

(57) Verfahren zur Herstellung von Azoxybenzol durch katalytische Hydrierung von Nitrobenzol mittels einer empirisch ermittelten Menge molekularem Wasserstoff in einer vorbereiteten Lösung eines Alkalialkoholates und mit Hilfe eines Edelmetallkatalysators bei Temperaturen zwischen 10 bis 120 °C und bei Druckverhältnissen von 0 bis 50 atü.

**Verfahren zur Herstellung von Azoxybenzol durch katalytische Hydrierung von Nitrobenzol mit molekularem Wasserstoff**

Die Erfindung betrifft ein Verfahren zur katalytischen Hydrierung von Nitrobenzol zu Azoxybenzol unter milden Reaktionsbedingungen und dessen einfache Isolierung in gut kristalliner Form.

Aus dem Stand der Technik ist es bekannt, Azoxybenzol durch Hydrierung von Nitrobenzol mit Hilfe von Alkali, Methanol, diversen Katalysatoren, Reduktionspromotoren und Zucker mit guten Ausbeuten und hohen Reinheiten zu erhalten. Es haftet aber allen bekannten Verfahren der Nachteil einer relativ grossen Kompliziertheit an, indem praktisch überall mittels Wasserdampfdestillation und anderen Einengungsprocederes gearbeitet werden muss. So wird u.a. in den nachstehenden Patentschriften und Literaturstellen folgendes beschrieben:

- DE-PS 486 598: Nitrobenzol wird mit wässriger Natronlauge versetzt und mit Hilfe von $Pb_2O$ reduziert. Der sich ergebende Niederschlag muss filtriert, gewaschen, extrahiert, durch Eindicken der Lösung wiedergewonnen und umkristallisiert werden.

- Nicholas Opolonick lehrt in einem Aufsatz in der Zeitschrift "Industrial and Engineering Chemistry" vom September 1935 die Reduzierung von Nitrobenzol mit Dextrose. Hier muss mittels Wasserdampfdestillation das Azoxybenzol vom parallel dazu gebildeten Anilin getrennt und aus Methanol rekristallisiert werden.

0017924

- 2 -

- US-PS 2,684,358, US-PS 2,794,046, US-PS 2,804,453: Alle drei
  Veröffentlichungen beschreiben den Einsatz eines Chinons als
  Reduktionspromotor, die Durchführung von Wasserdampfdestillation und anschliessend Phasentrennung und schliesslich die
  Aufarbeitung der bei der genannten Phasentrennung gebildeten
  "oberen öligen Phase" mittels HCl, Filtration und Trocknung.

Der vorliegenden Erfindung liegt die Aufgabe zugrunde, auf
möglichst oekonomische und umweltfreundliche Art Azoxybenzol
herzustellen und bietet gegenüber den beschriebenen Prozessen
die folgenden Vorteile:

- Keine Reaktionspartner, deren Folgeprodukte vernichtet
  werden müssen. Allfällige Nebenprodukte aus dem Nitrobenzol
  können zu Anilin weiterverarbeitet werden; Methanol nimmt
  bei den angewandten Bedingungen nicht an der Reaktion teil.
- Der Katalysator kann nach der Reaktion direkt aus der noch
  warmen Reaktionslösung abfiltriert werden.
- Das Azoxybenzol fällt aus der Reaktionslösung in gut kristalliner Form und hoher Reinheit aus. Deshalb keine Umkristallisation.
- Relativ tiefe Anlagekosten, bedingt durch die milden Reaktionsbedingungen.
- Kurze Hydrierzeiten, trotzdem niedrige Katalytkosten.

Die Lösung der Aufgabe der vorliegenden Erfindung erfolgt nach
der Lehre der Patentansprüche.

Das Prinzip vorliegender Erfindung ist das folgende:

Alkalihydroxid wird vorzugsweise in Methanol oder in Methanol/ Wasser gelöst. Statt Methanol können auch Aethanol, Propanol oder deren Mischungen mit Wasser verwendet werden. Als Alkoholate kommen Natrium-, Kalium- oder Lithiumalkoholate in Frage, wobei Natrium- oder Kaliumalkoholate bevorzugt werden.

Das Nitrobenzol wird darauf in der vorbereiteten Lösung in einem geeigneten Reaktor mit molekularem Wasserstoff hydriert. Pro Mol Nitrobenzol werden zweckmässig 0,3 bis 1,2 Mol Alkalihydroxid, gelöst in 200 bis 500 g Alkohol, zum Einsatz gebracht.

Als Katalysatoren werden Edelmetalle auf Träger oder Edelmetalloxide auf Träger verwendet. Solche Edelmetalle bzw. Edelmetalloxide sind Ruthenium, Rhodium, Palladium und Platin, bzw. deren Oxide.

Als Träger kommen in Frage Aktivkohle, $Al_2O_3$, $SiO_2$, $CaCO_3$ und Aluminiumsilikate. Vorzugsweise wird ein Katalysator aus Platin oder Palladium auf Aktivkohle eingesetzt.

Die Reaktionsbedingungen können, auch beeinflusst vom Lösungsmittel, in relativ grossen Bereichen von 10 bis 120°C und 0 bis 50 atü variiert werden. Bevorzugt werden 25 bis 50°C und 2 bis 10 atü.

Da die Hydrierung bei vorliegendem Verfahren ohne markanten Haltepunkt über die Azoxystufe hinaus weitergehen kann, wird zweckmässig so vorgegangen, dass nach einer empirisch ermittelten Menge reagiertem Wasserstoff die Reaktion gestoppt wird. Die Dauer der Hydrierung wird zweckmässig solange gewählt, dass die Endkonzentration an Nitrobenzol im Reaktionsgemisch im Bereich von 0,5 und 15% liegt, bevorzugt 1 bis 5%.

- 5 -

Statt den Wasserstoffverbrauch als Endpunktbestimmung zu verwenden, kann auch eine Gehaltsbestimmung "on-line" auf Nitrobenzol einfacher und genauer durchgeführt werden. Geeignet dazu sind gaschromatographische, potentiometrische und polarographische Bestimmungsmethoden.

Darauf wird der Katalyt vorteilhaft aus der noch über 40°C warmen Lösung abfiltriert und aus dem Filtrat das Azoxybenzol unter langsamem Abkühlen auskristallisiert. Nach der Filtration wird mit wenig Wasser nachgewaschen, um die Natronlauge zu entfernen.

Das Azoxybenzol liegt in schöner kristalliner Form vor und muss nur noch getrocknet werden, wenn bei der Auskristallisation darauf geachtet wird, dass sich dieses während dem Abkühlen nicht primär flüssig ausscheidet.

Es ist aber auch möglich, nach Abtrennung des Katalysators das Azoxybenzol durch fraktionierte Kristallisation über zwei bis drei Stufen zu gewinnen. Es gibt dann Produkte verschiedener Qualität, die nach Anforderung gemischt werden.

Das Filtrat kann nach der Rückgewinnung des Alkohols weiter verwertet werden. Da bei der Hydrierung von Nitrobenzol unter den angewandten Bedingungen als Nebenprodukt nur Anilin und dessen Vorstufen entstehen, können diese in einer zweiten Hydrierstufe quantitativ zu Anilin verarbeitet werden.

Beispiel 1

24,4 g NaOH wurden in 305 g Methanol gelöst und die Lösung zusammen mit 123,1 g Nitrobenzol und 150 mg Pt-Katalyt (5% Pt auf Aktivkohle) in einen 1,25 l Autoklaven gegeben. Der Autoklav war ausgerüstet mit Rührer, Thermoelement, Manometer, absolut dichtenden Ventilen, Vakuumpumpe und thermostatisiertem Wasserbad. Nachdem die Luft im absolut dichten Autoklav durch Stickstoff und der Stickstoff durch Wasserstoff ersetzt worden war, wurde bei schwachem Rühren auf 50°C aufgewärmt (bei schwachem Rühren findet noch praktisch keine Reaktion statt). Darauf wurde unter stärkerem Rühren und bei einer möglichst konstanten Temperatur von 50°C die Hydrierung durchgeführt. Der Wasserstoffverbrauch wurde kontinuierlich gemessen, indem man durch kurzes Oeffnen des Wasserstoffventils den Druck auf 10 atü steigen und darauf bei geschlossenem Ventil auf 8 atü fallen liess. Da die Reaktion stark exotherm ist und die Endpunktbestimmung über den Wasserstoffverbrauch erfolgte, musste die Temperatur neben der Wasserkühlung über die Rührgeschwindigkeit geregelt werden. (Da die Reaktion nicht sehr temperaturempfindlich ist, könnte bei einer anderen Endpunktbestimmungsmethode auf die Konstanhaltung der Temperatur verzichtet werden.) Nach dem gewünschten Wasserstoffverbrauch wurde nach einer Reaktionszeit von 105 Minuten die Reaktion durch Verlangsamung des Rührens gestoppt, vor dem Oeffnen des Reaktors der Wasserstoff durch Stickstoff ersetzt und der Katalyt aus der warmen Reaktionslösung abfiltriert.

_- 6 -_

Das Filtrat wurde darauf wieder auf ungefähr 50°C erwärmt, dann unter Rühren langsam auf 0°C abgekühlt. Die Abkühlung muss so erfolgen, dass sich das Azoxybenzol nicht flüssig ausscheidet. Nach der Filtration wurde das Kristallisat auf dem Filter mit wenig Wasser nachgewaschen und anschliessend bei 30°C/20 Torr auf Gewichtskonstanz getrocknet.

Ausbeute: 86,7 g 96,1%iges Azoxybenzol (entspricht 84,1% der Theorie, bezogen auf eingesetztes Nitrobenzol).

Die Hauptverunreinigung war Azobenzol.

1,4% des Nitrobenzols hatten sich nicht umgesetzt.

Beispiel 2

Wie Beispiel 1, aber mit den folgenden Abänderungen:

- 200 mg Pt-Katalyt (5% Pt auf Aktivkohle)
- Reaktionstemperatur 40°C
- Reaktionszeit 75 Minuten
- Endkonzentration Nitrobenzol 3,5%
- Zweimaliges Auskristallisieren ──→ 1. Kristallisat: kühlen bis auf 10°C,  2. Kristallisat: kühlen auf 0°C

Ausbeute: 1. Kristallisat 69,3 g 98,4%iges Azoxybenzol (entspricht 68,8% der Theorie) 2. Kristallisat 11,4 g 95,0%iges Azoxybenzol (entspricht 10,9% der Theorie) Beide Kristallisate gemischt 80,7 g 97,9%iges Azoxybenzol (entspricht 79,7% der Theorie).

- 7 -

Beispiel 3

Wie Beispiel 1, aber mit den folgenden Abänderungen:

- 100 mg Pd-Katalyt (5% Pd auf Aktivkohle)

- Reaktionstemperatur 40°C

- Reaktionszeit 47 Minuten

- Endkonzentration Nitrobenzol 3,7%

Ausbeute: 76,7 g 97,4%iges Azoxybenzol (entspricht 75,4%

   der Theorie).

- 1 -

Patentansprüche

1. Verfahren zur Herstellung von Azoxybenzol durch katalytische Hydrierung von Nitrobenzol, dadurch gekennzeichnet, dass Nitrobenzol, in eine vorbereitete Lösung eines Alkalialkoholates eingeführt, mit Hilfe eines auf Träger aufgebrachten Edelmetallkatalysators, mit molekularem Wasserstoff bei einer Temperatur zwischen 10 bis 120°C, bei Druckverhältnissen von 0 bis 50 atü hydriert wird und dass das nach Abfiltrieren des Katalysators erhaltene Endprodukt durch Auskristallisieren auf. Anhieb in grosser Reinheit ausfällt.

2. Verfahren nach Patentanspruch 1, dadurch gekennzeichnet, dass die vorbereitete Lösung eines Alkalialkoholates aus einem Alkalihydroxid in Methanol, Aethanol, Propanol oder deren Mischungen mit Wasser besteht.

3. Verfahren nach Patentansprüchen 1 und 2, dadurch gekennzeichnet, dass die Hydrierung mit molekularem Wasserstoff solange fortgesetzt wird, bis die Endkonzentration an Nitrobenzol im Reaktionsgemisch im Bereich von 0,5 bis 15% liegt.

4. Verfahren nach Patentansprüchen 1 und 2, dadurch gekennzeichnet, dass die Hydrierung mit molekularem Wasserstoff solange forgesetzt wird, bis die Endkonzentration an Nitrobenzol im Reaktionsgemisch im Bereich von 1 bis 5% liegt.

5. Verfahren nach Patentansprüchen 1 bis 4, dadurch gekennzeichnet, dass die Hydrierung bei Temperaturen von 25 bis
50°C durchgeführt wird.

6. Verfahren nach Patentansprüchen 1 bis 5, dadurch gekennzeichnet, dass die Hydrierung bei einem Wasserstoffdruck
von 2 bis 10 atü durchgeführt wird.